# EUROPEAN PATENT APPLICATION

(11) **EP 4 516 339 A1**
(43) Date of publication of application: **05.03.2025**
(21) Application number: 23854682.4
(22) Date of filing: 13.04.2023
(51) Int. Cl.: A61M 5/24, A61M 5/142, A61M 5/20

(54) **MEDICAL SOLUTION ADMINISTRATION DEVICE AND METHOD FOR MANUFACTURING MEDICAL SOLUTION ADMINISTRATION DEVICE**

(30) Priority: 18.08.2022 JP 2022130557
(71) Applicant: Terumo Kabushiki Kaisha, Tokyo 151-0072 (JP)
(72) Inventor: NAKANISHI, Masaru, Ashigarakami-gun, Kanagawa 259-0151 (JP)
(74) Representative: KIPA AB
(86) International application number: PCT/JP2023/014963
(87) International publication number: WO 2024/038642

(57) **Abstract**

In a liquid medicine administration device (10), a sterilization seal structure (22) includes a first connection seal portion (128) and a second connection seal portion (130). In an initial state, a drive unit (20) protrudes from a housing (12) in a direction opposite to a distal end direction of a syringe main body (54). In a state where the first connection seal portion (128) and the second connection seal portion (130) are opened, the drive unit (20) is pushed into the housing (12) in a distal end direction of a prefilled syringe (16) so that a protruding length of the drive unit (20) from the housing (12) is shortened, whereby a connection portion (92) is connected to a distal end portion of the prefilled syringe (16).

## Description

### Technical Field

The present invention relates to a liquid medicine administration device and a method for manufacturing the liquid medicine administration device.

### Background Art

For example, US 2020/0405951 A1 discloses a liquid medicine administration device including a prefilled syringe, a puncture unit, a housing, a drive unit, and a sterilization seal structure. The puncture unit includes a puncture needle capable of puncturing skin of a living body, a connection needle capable of penetrating a sealing member at a distal end portion of the prefilled syringe, and a needle coupling portion that couples the puncture needle and the connection needle to each other. The connection needle is separated from the sealing member in an initial state.

The housing accommodates the prefilled syringe, the puncture unit, and the drive unit. The sterilization seal structure includes a puncture seal portion for sealing the puncture needle, a first connection seal portion for sealing the connection needle, and a second connection seal portion for sealing the sealing member. The drive unit is fixed in the housing. The drive unit includes a motor, a gear mechanism, and the like that move the connection needle toward the sealing member in the housing.

In a case where such a liquid medicine administration device is used, after the sterilization seal structure (the puncture seal portion, the first connection seal portion, and the second connection seal portion) is removed, the motor is driven to cause the connection needle to penetrate the sealing member.

### Summary of Invention

In the liquid medicine administration device, in a case where the connection needle is separated from the sealing member in the initial state, a size of the liquid medicine administration device along an axial direction of the prefilled syringe increases. The liquid medicine administration device is desirably as compact as possible in size along the axial direction of the prefilled syringe at the time of use. However, in the conventional technology described above, the size of the liquid medicine administration device along the axial direction of the prefilled syringe is not shortened at the time of use. Furthermore, since the motor, the gear mechanism, and the like for moving the connection needle are required, the liquid medicine administration device becomes complicated.

An object of the present invention is to solve the problem described above.

(1) One aspect of the present invention is a liquid medicine administration device including: a prefilled syringe; a puncture unit including a puncture needle capable of puncturing skin of a living body, and a connection portion coupled to a proximal end of the puncture needle and connectable to a distal end portion of the prefilled syringe; a housing that accommodates the puncture unit and the prefilled syringe; a drive unit for pressing a gasket of the prefilled syringe in a distal end direction; and a sterilization seal structure for holding the puncture unit and the prefilled syringe in a sterilization state in an initial state, the connection portion being separated from the distal end portion of the prefilled syringe in the initial state, and the sterilization seal structure includes: a puncture seal portion for sealing the puncture needle; a first connection seal portion for sealing the connection portion; a second connection seal portion for sealing the distal end portion of the prefilled syringe; and an opening member including the first connection seal portion, the second connection seal portion, and an opening member main body, the opening member is attached to the housing such that the first connection seal portion and the second connection seal portion are openable, the prefilled syringe is fixed to the drive unit, the drive unit is slidably inserted into the housing together with the prefilled syringe in the distal end direction of the prefilled syringe, the drive unit protrudes from the housing in a direction opposite to the distal end direction of the prefilled syringe in the initial state, and by pushing, in a state where the first connection seal portion and the second connection seal portion are opened, the drive unit into the housing in the distal end direction of the prefilled syringe such that a protruding length of the drive unit from the housing is shortened, the connection portion is connected to the distal end portion of the prefilled syringe.
(2) In the liquid medicine administration device according to the item (1), the connection portion includes: a connection needle capable of penetrating a sealing member at the distal end portion of the prefilled syringe; and a needle coupling portion that couples a proximal end of the connection needle and a proximal end of the puncture needle to each other, the first connection seal portion seals the connection needle, the second connection seal portion seals the sealing member, and the connection needle penetrates the sealing member by pushing the drive unit into the housing.
(3) In the liquid medicine administration device according to the item (1) or (2), the opening member includes the puncture seal portion.
(4) In the liquid medicine administration device according to any one of the items (1) to (3), an adhesive member adherable to the skin of the living body is fixed to an outer surface of the housing, and the opening member is attached to the housing so as to cover at least a part of the adhesive member from outside.
(5) In the liquid medicine administration device according to the item (4), the housing includes: a bottom wall portion; and a pair of side wall portions protruding from both end portions of the bottom wall portion in a width direction orthogonal to an axial direction of the prefilled syringe, the adhesive member is fixed to an outer surface of the bottom wall portion of the housing and extends in a sheet shape, the opening member main body includes a base portion extending along shapes of the bottom wall portion and the pair of side wall portions of the housing, and the opening member is attached to the housing by engaging both end portions of the base portion with the pair of side wall portions.
(6) In the liquid medicine administration device according to the item (5), a first opening portion and a second opening portion are formed in the bottom wall portion of the housing, the first opening is a hole through which the puncture needle protrudes outward from the housing, the opening member main body includes an insertion portion to be inserted into the second opening, the insertion portion is positioned between the prefilled syringe and the connection portion in a state of being inserted into the second opening portion, the puncture seal portion is fixed to the base portion such that the first opening portion is sealed in the initial state, and the first connection seal portion is fixed to the insertion portion.
(7) In the liquid medicine administration device according to the item (6), the opening member main body is formed by integrally molding the base portion and the insertion portion.
(8) In the liquid medicine administration device according to the item (7), the opening member main body is configured by a resin material.
(9) In the liquid medicine administration device according to the item (8), the second connection seal portion is formed in a film shape and is joined to the opening member main body.
(10) In the liquid medicine administration device according to the item (9), the second connection seal portion extends in one direction, a slit through which the second connection seal portion is inserted is formed in the base portion, one end portion of the second connection seal portion is joined to the distal end portion of the prefilled syringe, and the other end portion of the second connection seal portion is joined to an outer surface of the base portion on a side opposite to a surface on which the insertion portion is provided.
(11) In the liquid medicine administration device according to the item (10), in the initial state, the second connection seal portion includes: a first extension portion that extends in a direction opposite to the second opening portion from the one end portion of the second connection seal portion; a folded portion positioned at an extension end portion of the first extension portion; and a second extension portion that extends from the folded portion toward the second opening portion.
(12) In the liquid medicine administration device according to any one of the items (1) to (11), each of the puncture seal portion and the first connection seal portion is configured by a silicone material.
(13) Another aspect of the present invention is a method for manufacturing the liquid medicine administration device according to any one of the items (1) to (12), the method including: a first sterilization process of mounting the opening member main body to which the puncture seal portion and the first connection seal portion are fixed to the housing, and sterilizing the housing, the puncture unit disposed inside the housing, the puncture seal portion, the first connection seal portion, and the opening member main body; a second sterilization process of sterilizing the prefilled syringe and the second connection seal portion in a state where the second connection seal portion is joined to the distal end portion of the prefilled syringe; a mounting process of mounting the prefilled syringe subjected to the second sterilization process to the drive unit; and an insertion process of inserting the prefilled syringe and the drive unit into the housing.

According to the present invention, the drive unit is pushed into the housing so that the protruding length of the drive unit from the housing is shortened, whereby the connection portion is connected to the distal end portion of the prefilled syringe. Therefore, a size of the liquid medicine administration device along the axial direction of the prefilled syringe can be made compact at the time of use. Furthermore, since the drive unit can be pushed into the housing manually, a motor, a gear mechanism, and the like for connecting the connection portion to the distal end portion of the prefilled syringe are unnecessary. Therefore, the liquid medicine administration device can be simplified.

### Brief Description of Drawings

Fig. 1 is a perspective view of a liquid medicine administration device according to an embodiment of the present invention.
Fig. 2 is an exploded perspective view of the liquid medicine administration device of Fig. 1**.**
Fig. 3 is an exploded perspective view of the liquid medicine administration device in a state where the liquid medicine administration device of Fig. 1 is turned upside down and a sterilization seal structure is removed from a housing.
Fig. 4 is a schematic plan view of the liquid medicine administration device in a state where a second cover member is removed.
Fig. 5 is a cross-sectional view taken along a line V-V of Fig. 4.
Fig. 6 is a schematic cross-sectional view taken along a line VI-VI of Fig. 4.
Fig. 7 is a schematic cross-sectional view taken along a line VII-VII of Fig. 4.
Fig. 8 is a perspective view of the sterilization seal structure.
Fig. 9 is a perspective view of the sterilization seal structure as viewed from a direction different from that in Fig. 8.
Fig. 10 is a flowchart illustrating an example of a method for manufacturing the liquid medicine administration device of Fig. 1**.**
Fig. 11 is an explanatory view of a first sterilization process of Fig. 10.
Fig. 12 is an explanatory view of a second sterilization process of Fig. 10.
Fig. 13 is a first explanatory view of a mounting process of Fig. 10.
Fig. 14 is a second explanatory view of the mounting process of Fig. 10.
Fig. 15 is an explanatory view of a joining process of Fig. 10.
Fig. 16 is a first use explanatory view of the liquid medicine administration device of Fig. 1.
Fig. 17 is a second use explanatory view of the liquid medicine administration device of Fig. 1.
Fig. 18A is a third use explanatory view of the liquid medicine administration device of Fig. 1. Fig. 18B is a fourth use explanatory view of the liquid medicine administration device of Fig. 1.
Fig. 19 is a fifth use explanatory view of the liquid medicine administration device of Fig. 1.
Fig. 20 is a sixth use explanatory view of the liquid medicine administration device of Fig. 1.

### Description of Embodiments

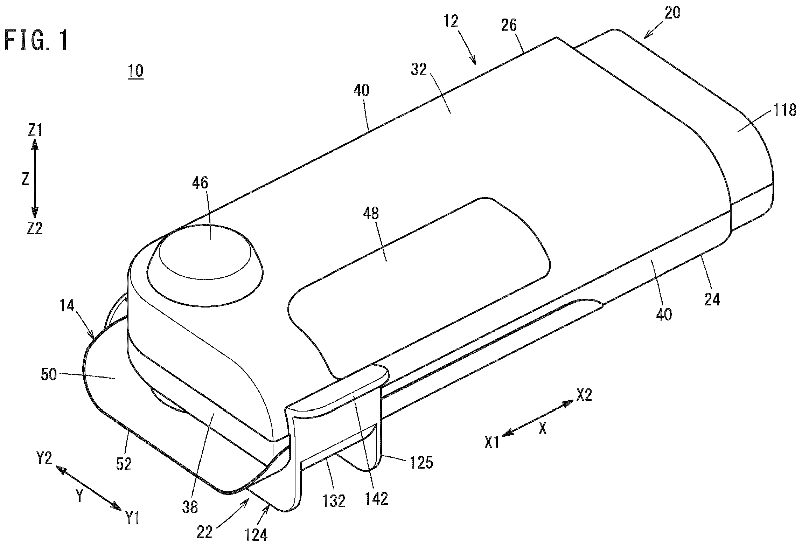

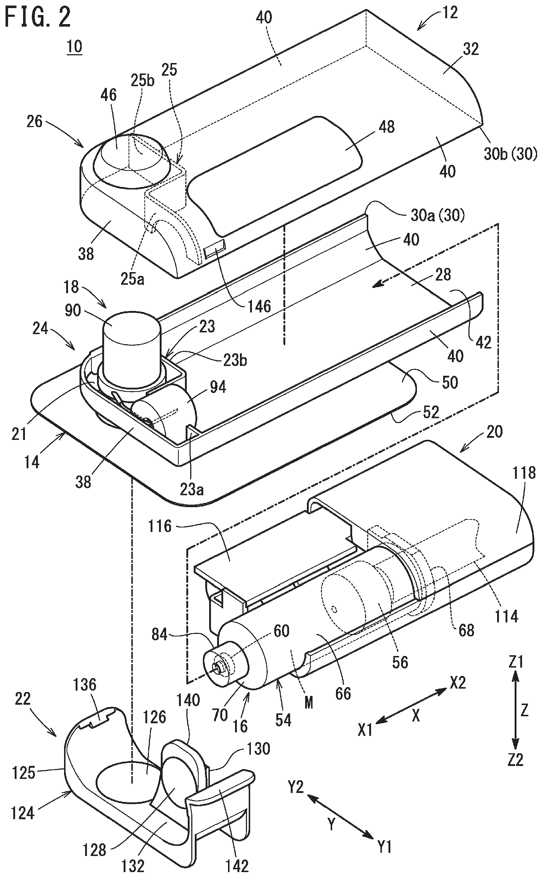

As illustrated in Figs. 1 and 2, a liquid medicine administration device 10 according to an embodiment of the present invention is used for administering a liquid medicine M into a living body. The liquid medicine administration device 10 continuously administers the liquid medicine M in a syringe main body 54 into the living body over a relatively long time (for example, about several minutes to several hours). The liquid medicine administration device 10 may intermittently administer the liquid medicine M into the living body. Examples of the liquid medicine M include protein preparations, narcotic analgesics, and diuretics.

As illustrated in Figs. 2 to 4, the liquid medicine administration device 10 includes a housing 12, an adhesive member 14, a prefilled syringe 16, a puncture unit 18, a drive unit 20, and a sterilization seal structure 22.

In Fig. 2, the housing 12 extends in an axial direction of the prefilled syringe 16 (arrow X direction). The housing 12 includes a first cover member 24 and a second cover member 26. The first cover member 24 has a bottom wall portion 28 of the housing 12. The first cover member 24 has a first peripheral wall portion 30a protruding in a direction (arrow Z1 direction) from an outer peripheral portion of the bottom wall portion 28 toward a ceiling portion 32 of the housing 12.

The second cover member 26 has the ceiling portion 32 of the housing 12. The second cover member 26 has a second peripheral wall portion 30b protruding in a direction (arrow Z2 direction) from an outer peripheral portion of the ceiling portion 32 toward the bottom wall portion 28 of the housing 12. The first peripheral wall portion 30a and the second peripheral wall portion 30b form a peripheral wall portion 30 of the housing 12 by engaging a protruding end portion of the first peripheral wall portion 30a and a protruding end portion of the second peripheral wall portion 30b with each other.

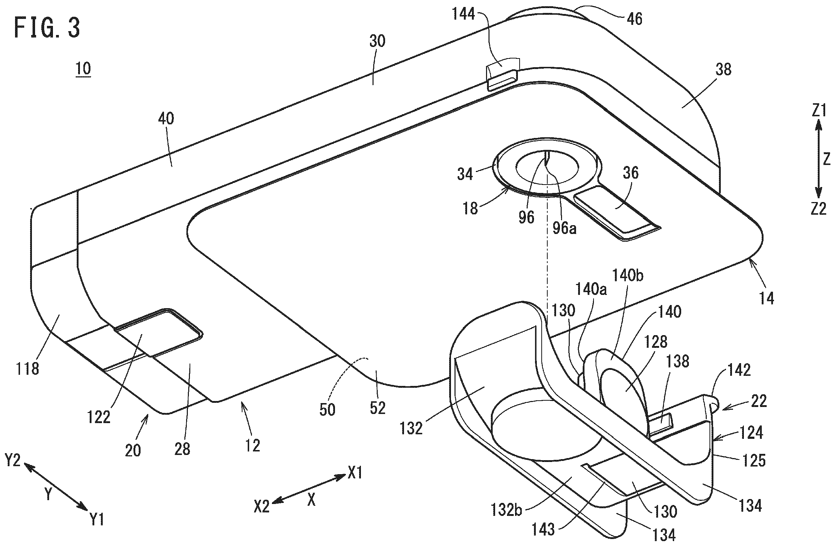

The prefilled syringe 16, the puncture unit 18, and the drive unit 20 are disposed on an inner surface of the bottom wall portion 28 of the housing 12. As illustrated in Fig. 3, the adhesive member 14 for being attached to skin of a living body is fixed to an outer surface of the bottom wall portion 28 of the housing 12. A first opening portion 34 and a second opening portion 36 are formed in the bottom wall portion 28 of the housing 12. The first opening portion 34 and the second opening portion 36 are positioned at one end portion (end portion in an arrow X1 direction) of the bottom wall portion 28. The first opening portion 34 and the second opening portion 36 are arranged in a width direction of the housing 12 (arrow Y direction).

The first opening portion 34 is a circular hole. The first opening portion 34 is a hole for causing a puncture needle 96 of the puncture unit 18 to protrude outward of the housing 12 toward skin of a living body. The second opening portion 36 is a slit extending in the arrow Y direction. The second opening portion 36 is a hole for inserting a part of the sterilization seal structure 22 from the outside to the inside of the housing 12.

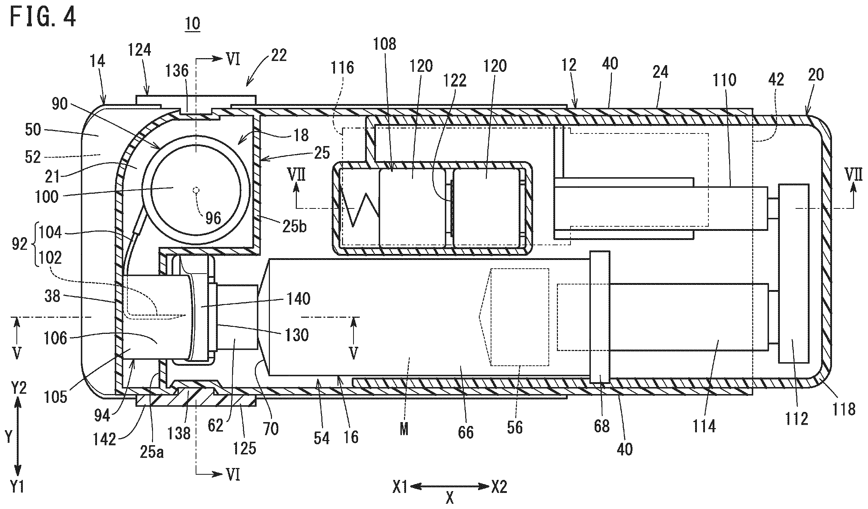

In Figs. 2 and 3, the peripheral wall portion 30 of the housing 12 includes an end wall portion 38 and a pair of side wall portions 40. The end wall portion 38 closes one end (end in the arrow X1 direction) of the housing 12. The pair of side wall portions 40 is positioned at both ends in the width direction of the housing 12. The other end portion (end in an arrow X2 direction) of the housing 12 is not closed by a wall portion. That is, an insertion port 42 for inserting the drive unit 20 is formed at the other end portion of the housing 12 (see Figs. 2 and 4).

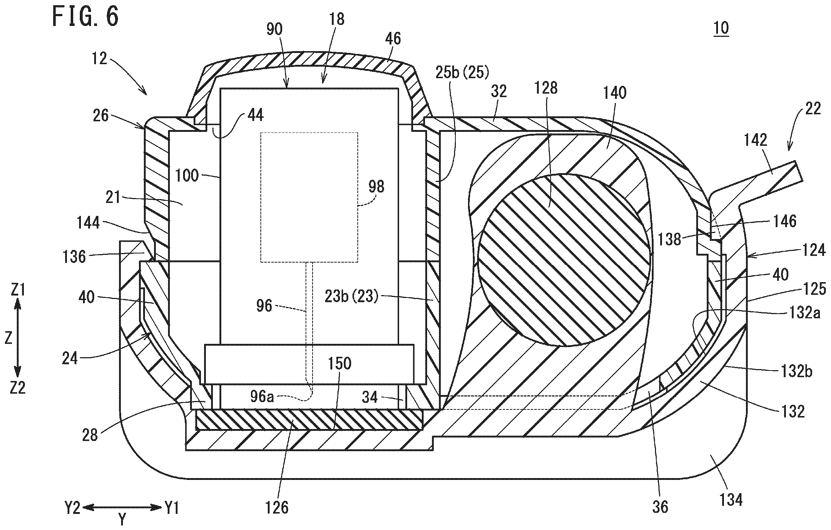

A through hole 44 is formed in the ceiling portion 32 of the housing 12 (see Fig. 6). In Figs. 1 and 2, the through hole 44 is covered with an operation cover 46 from the outside of the housing 12. The operation cover 46 has flexibility. The operation cover 46 is attached to the ceiling portion 32 of the housing 12 so that a human finger can push the operation cover 46 toward the through hole 44 to press the puncture unit 18.

The housing 12 is provided with a window 48 for visually recognizing the liquid medicine M in the prefilled syringe 16 positioned inside the housing 12. With this configuration, a remaining amount of the liquid medicine M in the prefilled syringe 16 can be easily checked.

As illustrated in Figs. 1 to 3, the adhesive member 14 is fixed to the outer surface of the bottom wall portion 28 of the housing 12 so as to protrude in the arrow X1 direction and the arrow Y direction from the housing 12. An end of the adhesive member 14 in the arrow X2 direction is positioned in the arrow X1 direction with respect to the other end of the housing 12. The adhesive member 14 includes an adhesive sheet 50 and a release sheet 52.

The adhesive sheet 50 is fixed to the outer surface of the bottom wall portion 28 of the housing 12. The adhesive sheet 50 has an adhesive surface (adhesion surface) that can be adhered to skin of a living body. The release sheet 52 is releasably adhered to the adhesive surface of the adhesive sheet 50 in an initial state of the liquid medicine administration device 10 (hereinafter simply referred to as an "initial state"). Note that a hole is formed in the adhesive member 14 such that the first opening portion 34 and the second opening portion 36 formed in the bottom wall portion 28 are not covered with the adhesive member 14 (see Fig. 3).

As illustrated in Figs. 2, 4, and 5, the prefilled syringe 16 includes the syringe main body 54, a gasket 56, a sealing member 60, and a distal end cap 62.

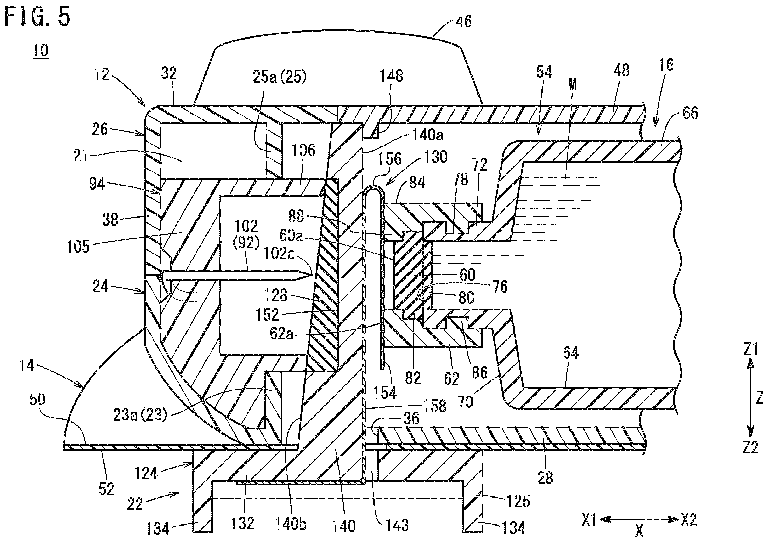

In Figs. 4 and 5, the syringe main body 54 is formed in a hollow cylindrical shape having a liquid medicine chamber 64 therein. The syringe main body 54 includes a body portion 66, a flange portion 68, a shoulder portion 70, and a nozzle 72.

As illustrated in Fig. 4, the body portion 66 extends in the axial direction of the prefilled syringe 16 (arrow X direction). An inner diameter and an outer diameter of the body portion 66 are constant (substantially constant) over an entire length of the body portion 66. A proximal end opening is formed at a proximal end of the body portion 66. The flange portion 68 protrudes radially outward from the proximal end (end in the arrow X2 direction) of the body portion 66 and extends annularly.

As illustrated in Fig. 5, a diameter of the shoulder portion 70 decreases from a distal end (end in the arrow X1 direction) of the body portion 66. The nozzle 72 protrudes in a distal end direction from a radially inner end of the shoulder portion 70. The nozzle 72 is formed in a tubular shape. A distal end opening 76 communicating with the liquid medicine chamber 64 is formed on a distal end of the nozzle 72. An annular recess 78 is formed on an outer peripheral surface of the nozzle 72. The liquid medicine M is filled in the syringe main body 54 in advance. The syringe main body 54 is preferably configured by a material having transparency.

In Fig. 4, the gasket 56 is slidably disposed inside the syringe main body 54. The gasket 56 is configured by, for example, an elastic resin material such as a rubber material or an elastomer material. An outer peripheral surface of the gasket 56 is in close contact with an inner peripheral surface of the body portion 66 of the syringe main body 54 in a liquid-tight manner.

As illustrated in Fig. 5, the sealing member 60 seals the distal end opening 76 of the nozzle 72 in a liquid-tight manner. The sealing member 60 is configured by an elastic resin material such as a rubber material or an elastomer material. The sealing member 60 includes a sealing main body 80 and an annular protrusion 82. The sealing main body 80 extends in a columnar shape along the axial direction of the prefilled syringe 16. One end portion of the sealing main body 80 is positioned outside the nozzle 72. The other end portion of the sealing main body 80 is inserted into the nozzle 72. An outer peripheral surface of the other end portion of the sealing main body 80 is in contact with an inner peripheral surface of the nozzle 72 in a liquid-tight manner.

The distal end cap 62 prevents the sealing member 60 from coming out of the nozzle 72. The distal end cap 62 is configured by, for example, a hard resin material. The distal end cap 62 includes a cap main body 84, a first locking protrusion 86, and a second locking protrusion 88.

The cap main body 84 is formed in an annular ring shape. The first locking protrusion 86 protrudes radially inward from an inner peripheral surface of the cap main body 84 and extends annularly. The first locking protrusion 86 is fitted into the annular recess 78 of the nozzle 72. The second locking protrusion 88 protrudes radially inward from an end of the cap main body 84 in the arrow X1 direction and extends annularly. The annular protrusion 82 is sandwiched between the second locking protrusion 88 and the nozzle 72.

As illustrated in Figs. 2 and 4, the puncture unit 18 includes a puncture mechanism 90, a connection portion 92, and a support portion 94. In Fig. 6, the puncture mechanism 90 includes the puncture needle 96, a needle hub 98, and a hub support portion 100. The puncture needle 96 has a needle tip 96a capable of puncturing skin of a living body. The puncture needle 96 extends in a thickness direction of the housing 12 (arrow Z direction). The puncture needle 96 is formed in a tubular shape. The needle hub 98 supports a proximal end portion of the puncture needle 96.

The hub support portion 100 movably supports the needle hub 98. The hub support portion 100 is attached to the bottom wall portion 28 of the housing 12. An end portion of the hub support portion 100 in the arrow Z1 direction is positioned in the through hole 44 of the ceiling portion 32 of the housing 12.

The hub support portion 100 supports the needle hub 98 so as to be movable from an initial position (position in Fig. 6) where the needle tip 96a of the puncture needle 96 is positioned in the housing 12 to a protruding position where the needle tip 96a is positioned outside the housing 12. The needle hub 98 moves from the initial position to the protruding position when the hub support portion 100 is pressed in the arrow Z2 direction via the operation cover 46. The needle hub 98 is held at the protruding position by being biased in the arrow Z2 direction by a biasing member (for example, a compression coil spring) not illustrated in a state of being positioned at the protruding position.

As illustrated in Figs. 4 and 5, the connection portion 92 includes a connection needle 102 and a needle coupling portion 104. The connection needle 102 has a needle tip 102a that can penetrate the sealing member 60. The connection needle 102 is formed in a tubular shape. The connection needle 102 extends along the axial direction of the prefilled syringe 16 (arrow X direction). The needle tip 102a of the connection needle 102 faces the arrow X2 direction. In the initial state, the needle tip 102a of the connection needle 102 is separated from the sealing member 60.

In Fig. 4, the needle coupling portion 104 couples a proximal end of the connection needle 102 and the proximal end of the puncture needle 96 to each other. The needle coupling portion 104 is a flexible tube that communicates a lumen of the connection needle 102 and a lumen of the puncture needle 96 with each other.

A support portion 94 is positioned in an arrow Y1 direction of the puncture mechanism 90. In other words, the puncture mechanism 90 and the support portion 94 are arranged in the width direction of the housing 12. As illustrated in Fig. 5, the support portion 94 includes a support main body 105 and a cylindrical portion 106. The support main body 105 is disposed in the housing 12 in a state of supporting the proximal end portion of the connection needle 102.

The cylindrical portion 106 protrudes from the support main body 105 in the arrow X2 direction. The cylindrical portion 106 covers an outer peripheral surface of the connection needle 102. An axis of the cylindrical portion 106 is positioned on an axis of the connection needle 102. A protruding end of the cylindrical portion 106 extends so as to be inclined in the arrow X1 direction toward the arrow Z2 direction. The inside of the cylindrical portion 106 is formed in a size into which a distal end portion (distal end cap 62) of the prefilled syringe 16 can be inserted.

As illustrated in Figs. 2 and 4 to 6, the puncture mechanism 90 is disposed in a sealed space 21 formed in the housing 12. The sealed space 21 is surrounded by the bottom wall portion 28, a part of the first peripheral wall portion 30a, a first partition wall portion 23, the ceiling portion 32, a part of the second peripheral wall portion 30b, a second partition wall portion 25, and the operation cover 46. An end surface of the first peripheral wall portion 30a in the arrow Z1 direction is welded to an end surface of the second peripheral wall portion 30b in the arrow Z2 direction.

In Figs. 2 and 5, the first partition wall portion 23 protrudes from the bottom wall portion 28 toward the ceiling portion 32. The first partition wall portion 23 extends in a width direction of the first cover member 24 so as to divide an internal space of the first cover member 24 in the arrow X direction. The first partition wall portion 23 includes a first support wall portion 23a and a first enclosure portion 23b.

The first support wall portion 23a extends from a side wall portion of the first cover member 24 in the arrow Y1 direction to a central portion of the first cover member 24 toward the width direction in an arrow Y2 direction. The first support wall portion 23a supports the cylindrical portion 106 from the arrow Z2 direction. The first support wall portion 23a is welded to an outer peripheral surface of the cylindrical portion 106. The first enclosure portion 23b extends from an end portion of the first support wall portion 23a in the arrow Y2 direction to a side wall portion of the first cover member 24 in the arrow Y2 direction so as to cover the puncture mechanism 90 from the arrow Y1 direction and the arrow X2 direction.

In Figs. 2 and 4, the second partition wall portion 25 protrudes from the ceiling portion 32 toward the bottom wall portion 28. The second partition wall portion 25 extends in a width direction of the second cover member 26 so as to divide an internal space of the second cover member 26 in the arrow X direction. The second partition wall portion 25 includes a second support wall portion 25a and a second enclosure portion 25b.

The second support wall portion 25a extends from a side wall portion of the second cover member 26 in the arrow Y1 direction to a central portion of the second cover member 26 in the width direction toward the arrow Y2 direction. The second support wall portion 25a supports the cylindrical portion 106 from the arrow Z1 direction. The second support wall portion 25a is welded to the outer peripheral surface of the cylindrical portion 106. The second enclosure portion 25b extends from an end portion of the second support wall portion 25a in the arrow Y2 direction to a side wall portion of the second cover member 26 in the arrow Y2 direction so as to cover the puncture mechanism 90 from the arrow Y1 direction and the arrow X2 direction. A protruding end surface (end surface in the arrow Z1 direction) of the first partition wall portion 23 is welded to a protruding end surface (end surface in the arrow Z2 direction) of the second partition wall portion 25. An outer peripheral edge portion of the operation cover 46 is welded to the ceiling portion 32 of the second cover member 26 (see Fig. 6).

As illustrated in Figs. 2 and 4, the drive unit 20 presses the gasket 56 in the distal end direction. The drive unit 20 is slidably inserted into the housing 12 together with the prefilled syringe 16 in a distal end direction of the prefilled syringe 16 (arrow X1 direction).

In Fig. 4, the drive unit 20 includes a power supply unit 108, a motor 110, a power transmission mechanism 112, a plunger 114, a control unit 116, and a drive cover portion 118. The power supply unit 108 supplies power to the motor 110 and the control unit 116. The power supply unit 108 is positioned on a side (arrow Y2 direction) of the body portion 66 of the syringe main body 54. The power supply unit 108 is disposed in the arrow X2 direction of the puncture mechanism 90. The power supply unit 108 includes, for example, a plurality of (two in the example of Fig. 4) batteries 120 disposed in series in the arrow X direction. The battery 120 may be either a primary battery or a secondary battery. The power supply unit 108 may have one or three or more batteries 120.

The motor 110 is positioned in the arrow X2 direction of the batteries 120. The power transmission mechanism 112 transmits a rotational driving force of the motor 110 to the plunger 114. The power transmission mechanism 112 includes, for example, a plurality of gears. The plunger 114 extends along the axial direction of the prefilled syringe 16. The plunger 114 presses the gasket 56 in the arrow X1 direction. A distal end portion of the plunger 114 is inserted into the syringe main body 54.

For example, the plunger 114 extends toward the gasket 56 (in the arrow X1 direction) by the rotational driving force transmitted from the power transmission mechanism 112 to the plunger 114. The control unit 116 controls an operation of the motor 110. The plunger 114 and the motor 110 are arranged at an interval in the width direction of the housing 12.

As illustrated in Figs. 2 and 4, the drive cover portion 118 integrally supports the power supply unit 108, the motor 110, the power transmission mechanism 112, the plunger 114, and the control unit 116. The prefilled syringe 16 is fixed to the drive cover portion 118.

In Figs. 1 and 4, in the initial state of the liquid medicine administration device 10, the drive unit 20 protrudes from the housing 12 in a direction opposite to the distal end direction of the prefilled syringe 16 (arrow X2 direction). In other words, in the initial state, an end portion of the drive unit 20 in the arrow X2 direction is positioned in the arrow X2 direction with respect to the other end of the housing 12.

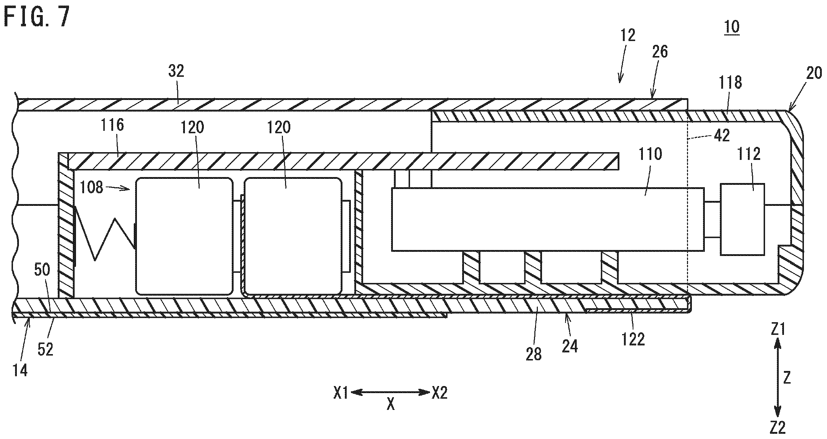

As illustrated in Fig. 7, in the initial state, an insulating film 122 is attached to the drive unit 20. In the initial state, the insulating film 122 blocks power supply from the power supply unit 108 to the motor 110 and the control unit 116. The insulating film 122 extends in one direction. In the present embodiment, one end portion of insulating film 122 is inserted between the two batteries 120 disposed in series. Note that the one end portion of the insulating film 122 may be inserted between the battery 120 and a positive terminal or between the battery 120 and a negative terminal.

The insulating film 122 extends from the power supply unit 108 to the insertion port 42 at the other end of the housing 12 through between the drive cover portion 118 and the inner surface of the bottom wall portion 28 of the housing 12. The insulating film 122 is folded back in the arrow X1 direction at a position of the other end of the housing 12. The other end portion of the insulating film 122 is joined (fixed) to the outer surface of the bottom wall portion 28 of the housing 12 (see Fig. 3).

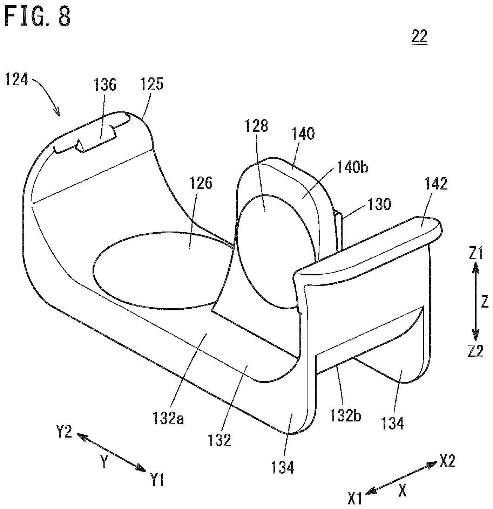

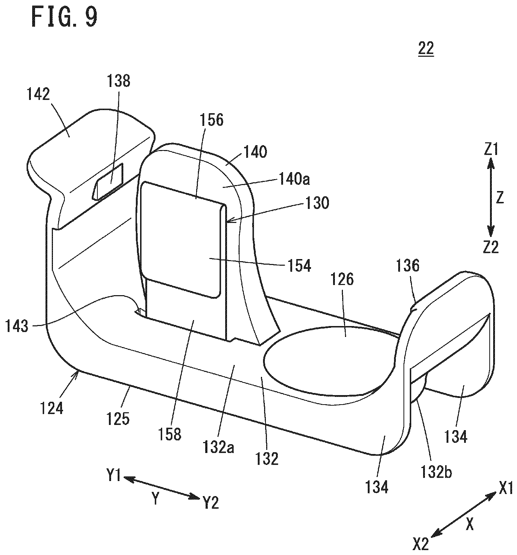

As illustrated in Figs. 8 and 9, the sterilization seal structure 22 holds the puncture unit 18 and the prefilled syringe 16 in a sterilization state in the initial state. The sterilization seal structure 22 includes an opening member 124. The opening member 124 includes an opening member main body 125, a puncture seal portion 126, a first connection seal portion 128, and a second connection seal portion 130. The opening member main body 125, the puncture seal portion 126, and the first connection seal portion 128 are integrally configured by two-color molding using a resin material.

The opening member main body 125 is configured by a hard resin material. Examples of the constituent material of the opening member main body 125 include, but are not limited to, polycarbonate. The opening member main body 125 is attachable to and detachable from the housing 12. In the initial state of the liquid medicine administration device 10, the opening member main body 125 is mounted to the housing 12 so as to cover at least a part of the adhesive member 14 from the outside (see Fig. 1).

In Figs. 3, 6, 8, and 9, the opening member main body 125 includes a base portion 132, a pair of ribs 134, a first engagement portion 136, a second engagement portion 138, an insertion portion 140, and an opening operation portion 142. As illustrated in Fig. 6, the base portion 132 extends in a plate shape along shapes of the bottom wall portion 28 and the pair of side wall portions 40 of the housing 12 in a state where the opening member main body 125 is mounted to the housing 12.

The base portion 132 includes an inner surface 132a and an outer surface 132b. The inner surface 132a of the base portion 132 is a surface facing the housing 12 in a state where the opening member 124 is mounted to the housing 12 (hereinafter may be simply referred to as a "mounted state"). The outer surface 132b of the base portion 132 is a surface facing a direction opposite to the inner surface 132a. In Fig. 3, a width (length in the arrow X direction) of the base portion 132 is wider than a diameter of the first opening portion 34 of the housing 12. A slit 143 through which the second connection seal portion 130 is inserted is formed in the base portion 132.

As illustrated in Figs. 3, 8, and 9, the pair of ribs 134 extends outward from both end portions in a width direction of the base portion 132. The pair of ribs 134 extends in an extending direction of the base portion 132.

In Fig. 6, the first engagement portion 136 is provided at one end portion of the base portion 132 in the extending direction. The first engagement portion 136 engages with a third engagement portion 144 formed on an outer surface of the side wall portion 40 of the housing 12 in the mounted state. The first engagement portion 136 and the third engagement portion 144 are configured such that the opening member 124 is rotatable about the first engagement portion 136. The first engagement portion 136 is a protrusion, and the third engagement portion 144 is a recess. Note that the first engagement portion 136 may be a recess, and the third engagement portion 144 may be a protrusion. The first engagement portion 136 is detachable from the third engagement portion 144.

The second engagement portion 138 is provided at the other end portion of the base portion 132 in the extending direction. The second engagement portion 138 engages with a fourth engagement portion 146 formed on the outer surface of the side wall portion 40 of the housing 12 in the mounted state. The second engagement portion 138 and the fourth engagement portion 146 are configured such that the engagement of the second engagement portion 138 and the fourth engagement portion 146 is released by operating the opening operation portion 142. The second engagement portion 138 is a protrusion, and the fourth engagement portion 146 is a recess. Note that the second engagement portion 138 may be a recess, and the fourth engagement portion 146 may be a protrusion.

As illustrated in Figs. 6, 8, and 9, the opening operation portion 142 extends from the other end portion of the base portion 132 in a direction opposite to the one end portion of the base portion 132. The opening operation portion 142 has a size and a shape that are easily operated by a human finger. The opening operation portion 142 extends from the other end portion of the base portion 132 so as to be inclined in the arrow Z1 direction toward the arrow Y1 direction.

The insertion portion 140 protrudes from a portion of the inner surface 132a of the base portion 132 facing the adhesive member 14 (the bottom wall portion 28 of the housing 12) in the mounted state. The insertion portion 140 is formed in a flat plate shape. As illustrated in Fig. 5, the insertion portion 140 is inserted into the second opening portion 36 of the housing 12 in the mounted state. The insertion portion 140 includes a first surface 140a and a second surface 140b.

The first surface 140a of the insertion portion 140 faces the prefilled syringe 16 in the mounted state. The first surface 140a is orthogonal to an axial direction of the syringe main body 54 (arrow X direction) in the mounted state. The first surface 140a is close to the distal end of the prefilled syringe 16. The first surface 140a is continuous with an inner surface of the slit 143 formed in the base portion 132. The first surface 140a abuts on a protrusion 148 protruding from an inner surface of the window 48 of the housing 12 in the mounted state. That is, movement of the insertion portion 140 in the arrow X2 direction relative to the housing 12 in the mounted state is restricted by the protrusion 148.

The second surface 140b of the insertion portion 140 faces the connection portion 92 in the mounted state. The second surface 140b is an inclined surface inclined in a direction in which the first surface 140a faces (arrow X2 direction) toward a protruding direction of the insertion portion 140 (arrow Z1 direction). The insertion portion 140 also functions as a stopper that blocks movement of the prefilled syringe 16 and the drive unit 20 in the arrow X1 direction relative to the housing 12 in the initial state.

As illustrated in Fig. 6, the puncture seal portion 126 seals the puncture needle 96 in the initial state. The puncture seal portion 126 is configured by a soft resin material. Examples of the constituent material of the puncture seal portion 126 include, but are not limited to, silicone. In Figs. 8 and 9, the puncture seal portion 126 is formed in a circular shape. The puncture seal portion 126 is positioned in a portion of the inner surface 132a of the base portion 132 adjacent to the insertion portion 140.

In Fig. 6, the puncture seal portion 126 is disposed in a recess 150 formed in the inner surface 132a of the base portion 132. The puncture seal portion 126 is fixed to the base portion 132. A diameter of the puncture seal portion 126 is larger than the diameter of the first opening portion 34 of the housing 12. The puncture seal portion 126 seals the first opening portion 34 in the initial state. In other words, in the initial state, the puncture seal portion 126 is in contact with a portion adjacent to the first opening portion 34 (a portion extending so as to surround the first opening portion 34) in the outer surface of the bottom wall portion 28 in a liquid-tight and gas-tight manner. The puncture needle 96 is sealed by bringing the puncture seal portion 126 into contact with the portion adjacent to the first opening portion 34 in the outer surface of the bottom wall portion 28 in a liquid-tight and gas-tight manner in a state where the puncture mechanism 90 is disposed in the sealed space 21 of the housing 12.

In Fig. 5, the first connection seal portion 128 seals the connection portion 92 (connection needle 102) in the initial state. The first connection seal portion 128 is configured by a constituent material similar to that of the puncture seal portion 126. The first connection seal portion 128 is formed in a circular shape (see Fig. 8).

The first connection seal portion 128 is disposed in a recess 152 formed on the second surface 140b of the insertion portion 140. The first connection seal portion 128 is fixed to the insertion portion 140. A diameter of the first connection seal portion 128 is larger than a diameter of the cylindrical portion 106. An outer surface of the first connection seal portion 128 exposed from the insertion portion 140 is inclined in the direction in which the first surface 140a faces (arrow X2 direction) toward the protruding direction of the insertion portion 140 (arrow Z1 direction). The outer surface of the first connection seal portion 128 is in contact with the protruding end of the cylindrical portion 106 in a liquid-tight and gas-tight manner in the mounted state.

The second connection seal portion 130 seals the distal end portion of the prefilled syringe 16 in the initial state. The second connection seal portion 130 extends in one direction and is formed in a film shape. Examples of a constituent material of the second connection seal portion 130 include, but are not limited to, polyethylene terephthalate (PET).

A distal end surface 62a of the distal end cap 62 is joined to one end portion of the second connection seal portion 130 so that a distal end surface 60a of the sealing member 60 is sealed. The second connection seal portion 130 is not joined to the distal end surface 60a of the sealing member 60. The one end portion of the second connection seal portion 130 may be joined to the distal end surface 60a of the sealing member 60 in a state where the distal end surface 60a of the sealing member 60 protrudes in the arrow X1 direction from the distal end surface 62a of the distal end cap 62. The other end portion of the second connection seal portion 130 is joined to the outer surface 132b of the base portion 132.

Examples of a method for joining the second connection seal portion 130 to the distal end portion of the prefilled syringe 16 include welding, but are not limited thereto, and adhesion using an adhesive or the like may be used. Furthermore, examples of a method for joining the second connection seal portion 130 to the base portion 132 include welding, but are not limited thereto, and adhesion using an adhesive or the like may be used.

The second connection seal portion 130 includes a first extension portion 154, a folded portion 156, and a second extension portion 158. The first extension portion 154 extends from the one end portion of the second connection seal portion 130 in a direction opposite to the second opening portion 36 (arrow Z1 direction). The folded portion 156 is positioned at an extension end portion (end portion in the arrow Z1 direction) of the first extension portion 154.

In Fig. 5, the folded portion 156 has a shape obtained by turning a U shape upside down. The second extension portion 158 extends from the folded portion 156 to the other end portion of the second connection seal portion 130. The second extension portion 158 is inserted through the second opening portion 36 and the slit 143. The second extension portion 158 is in contact with or close to the first surface 140a of the insertion portion 140.

Next, an example of a method for manufacturing (method for assembling) the liquid medicine administration device 10 will be described. Note that, in the following description, it is assumed that the adhesive member 14 and the operation cover 46 are attached to the housing 12 in advance.

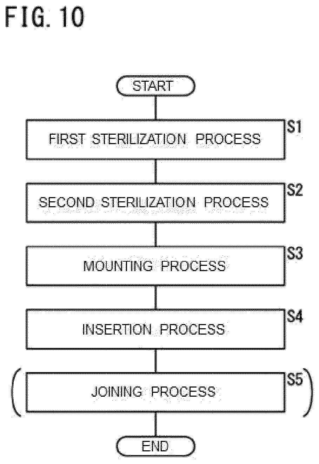

As illustrated in Fig. 10, the method for manufacturing the liquid medicine administration device 10 includes, for example, a first sterilization process, a second sterilization process, a mounting process, an insertion process, and a joining process. Note that the joining process is not an essential process in the present embodiment, and can be omitted.

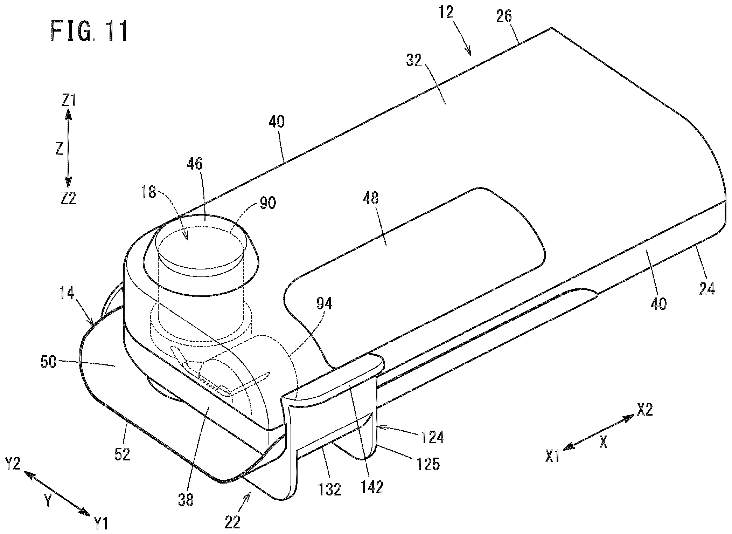

First, in the first sterilization process (step S1), as illustrated in Fig. 11, the housing 12, the puncture unit 18 disposed inside the housing 12, and the opening member main body 125 to which the puncture seal portion 126 and the first connection seal portion 128 are fixed are sterilized. In the first sterilization process, the opening member main body 125 is mounted to the housing 12. Note that, in the first sterilization process, the second connection seal portion 130 is not attached to the opening member main body 125. In the first sterilization process, for example, autoclave sterilization is performed. Note that a sterilization method can be appropriately set.

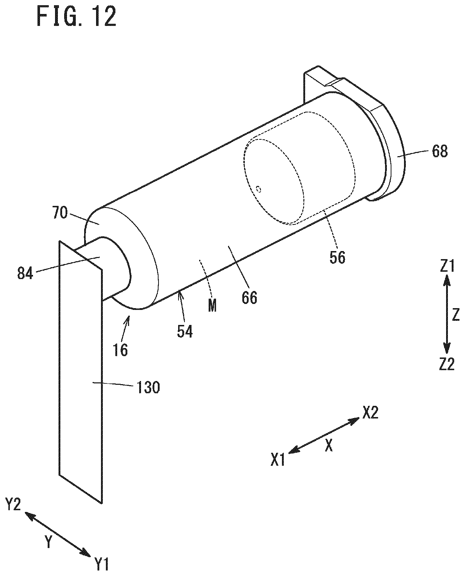

Furthermore, in the second sterilization process (step S2 in Fig. 10), as illustrated in Fig. 12, the prefilled syringe 16 and the second connection seal portion 130 are sterilized in a state where the one end portion of the second connection seal portion 130 is joined to the distal end portion of the prefilled syringe 16. In the second sterilization process, for example, autoclave sterilization is performed. Note that a sterilization method can be appropriately set.

Subsequently, in the mounting process (step S3 in Fig. 10), the prefilled syringe 16 subjected to the second sterilization process is mounted to the drive unit 20.

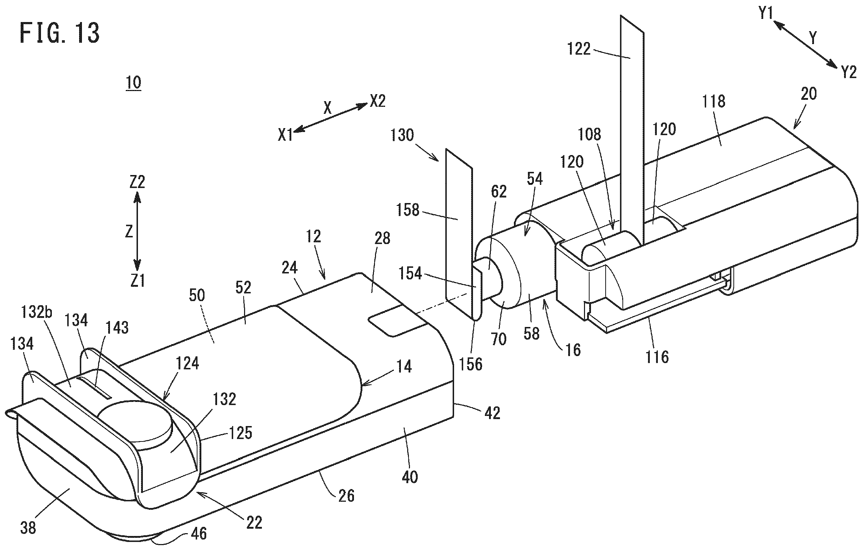

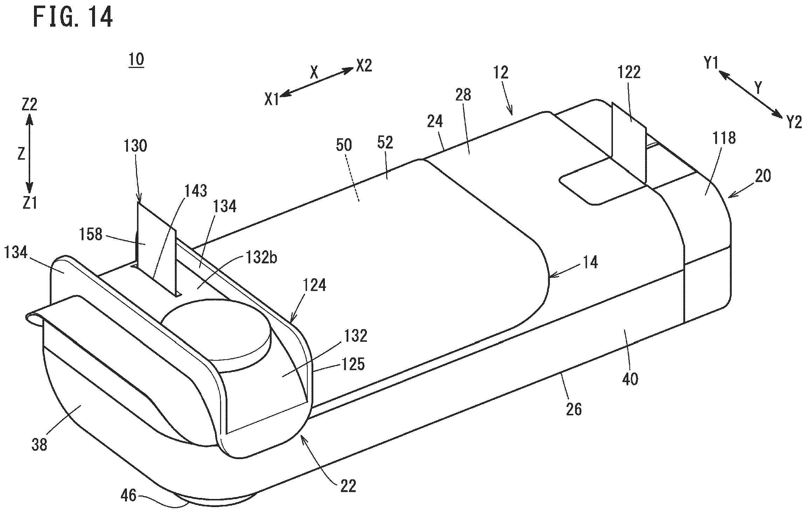

Thereafter, in the insertion process (step S4 in Fig. 10), as illustrated in Fig. 13, the prefilled syringe 16 and the drive unit 20 are inserted into the housing 12 from the insertion port 42 of the housing 12. At this time, as illustrated in Fig. 14, an intermediate portion of the second connection seal portion 130 is folded back and passed through the second opening portion 36 (see Fig. 5) of the housing 12 and the slit 143 of the opening member 124.

In this state, the drive unit 20 protrudes in the arrow X2 direction from the other end of the housing 12, and the other end portion of the insulating film 122 protrudes in the arrow X2 direction from the other end of the housing 12.

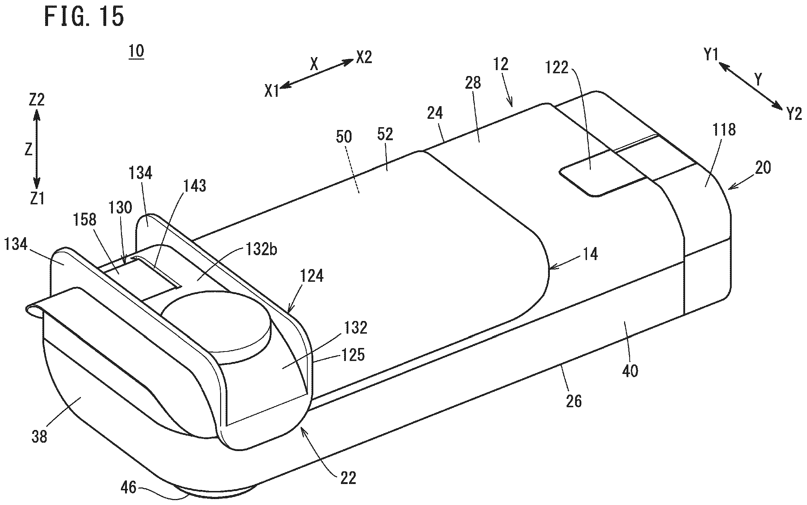

Thereafter, in the joining process (step S5 in Fig. 10), as illustrated in Fig. 15, the second connection seal portion 130 is joined (welded) to an outer surface of the opening member main body 125 (the outer surface 132b of the base portion 132), and the other end portion of the insulating film 122 is folded back in the arrow X1 direction and joined (welded) to the outer surface of the bottom wall portion 28 of the housing 12. With this configuration, the manufacture of the liquid medicine administration device 10 is completed. Note that, in the present embodiment, an extra portion of the second connection seal portion 130 may be cut off so that a length of a portion of the second connection seal portion 130 positioned outside the housing 12 becomes an appropriate length. In this case, workability is improved, and external appearance of the liquid medicine administration device 10 can be made uniform.

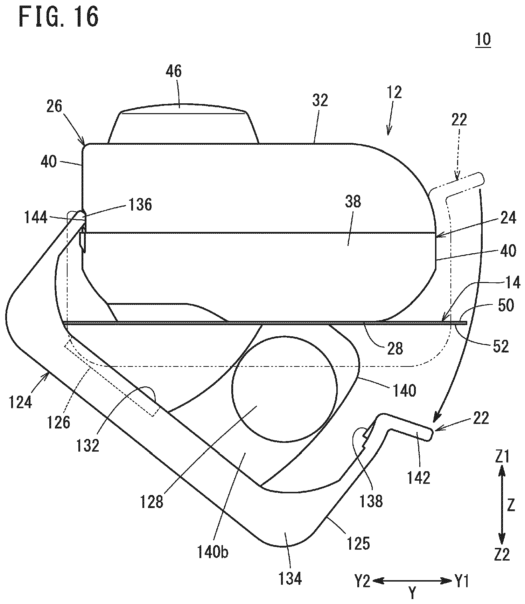

Next, a method for using the liquid medicine administration device 10 will be described. In the case of using the liquid medicine administration device 10, first, as illustrated in Fig. 16, a user removes the opening member 124 from the housing 12. Specifically, the user presses the opening operation portion 142 in the arrow Z2 direction with fingers. Then, since the second engagement portion 138 is disengaged from the fourth engagement portion 146 (see Fig. 2), the opening member 124 rotates about the first engagement portion 136. As a result, the puncture seal portion 126 is separated from the outer surface of the bottom wall portion 28 of the housing 12. That is, sealing of the puncture needle 96 by the puncture seal portion 126 is opened.

Furthermore, the insertion portion 140 comes out of the second opening portion 36. As a result, the first connection seal portion 128 is separated from the protruding end of the cylindrical portion 106. That is, sealing of the connection needle 102 by the first connection seal portion 128 is opened.

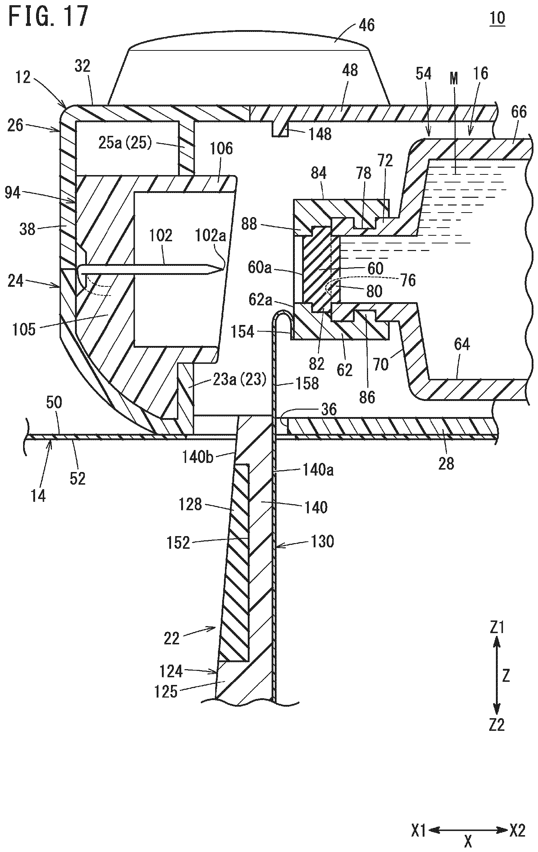

At this time, as illustrated in Fig. 17, the second connection seal portion 130 is pulled in the arrow Z2 direction. As a result, a joint surface of the second connection seal portion 130 joined to the distal end portion of the prefilled syringe 16 is gradually peeled off from the arrow Z1 direction toward the arrow Z2 direction. Then, the second connection seal portion 130 is completely removed from the distal end portion of the prefilled syringe 16, whereby sealing of the distal end portion of the prefilled syringe 16 by the second connection seal portion 130 is opened. Thereafter, the first engagement portion 136 of the opening member 124 is removed from the third engagement portion 144 of the housing 12. As a result, the opening member 124 is completely separated from the housing 12.

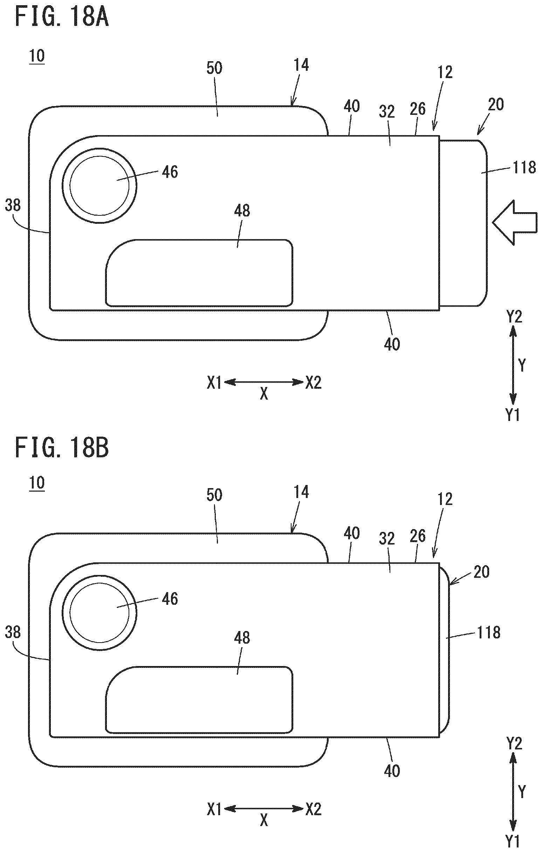

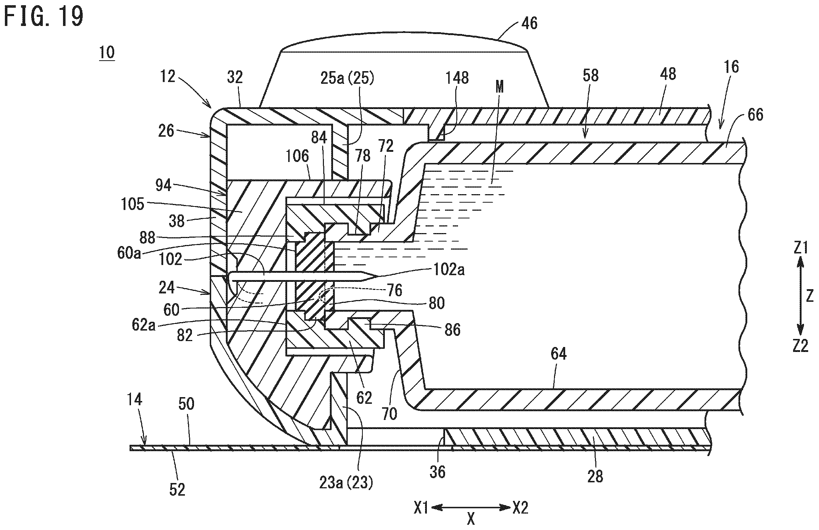

Subsequently, as illustrated in Figs. 18A and 18B, the user pushes the drive unit 20 into the housing 12 in the arrow X1 direction so that a protruding length of the drive unit 20 from the housing 12 is shortened. Then, as illustrated in Fig. 19, the distal end portion of the prefilled syringe 16 is inserted into the cylindrical portion 106, and the connection needle 102 penetrates the sealing member 60. As a result, the needle tip 102a of the connection needle 102 is positioned in the nozzle 72.

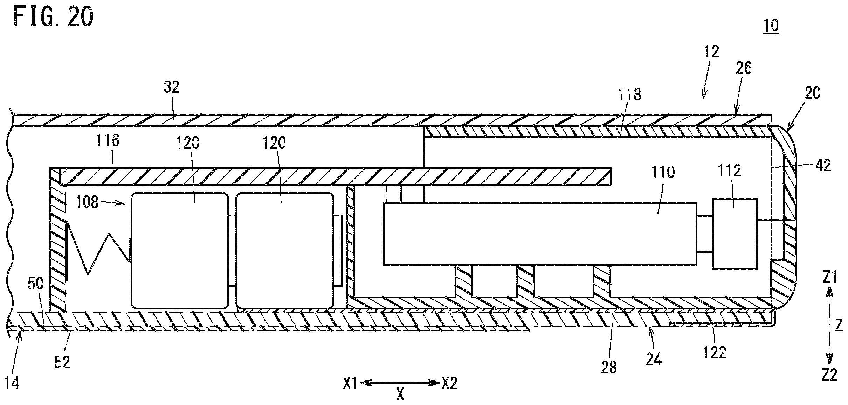

Furthermore, as illustrated in Fig. 20, when the one end portion of insulating film 122 comes out of between the two batteries 120, the batteries 120 are electrically connected to each other. As a result, the motor 110 can be driven.

Subsequently, the release sheet 52 is peeled off from the adhesive sheet 50, and the adhesive sheet 50 is adhered to skin of a living body. Thereafter, the user presses the operation cover 46 to puncture the skin with the puncture needle 96. Next, the user operates a mobile terminal (not illustrated) or the like capable of communicating with the control unit 116 to rotate the motor 110. The rotational driving force of the motor 110 is transmitted to the plunger 114 via the power transmission mechanism 112. Then, since the plunger 114 presses the gasket 56 in the distal end direction of the prefilled syringe 16, the liquid medicine M filled in the syringe main body 54 is administered into the skin via the connection needle 102, the needle coupling portion 104, and the puncture needle 96.

The present embodiment has the following effects.

According to the present embodiment, the drive unit 20 is pushed into the housing 12 so that the protruding length of the drive unit 20 from the housing 12 is shortened, whereby the connection portion 92 is connected to the distal end portion of the prefilled syringe 16. Therefore, a size of the liquid medicine administration device 10 along the axial direction of the prefilled syringe 16 can be made compact at the time of use. Furthermore, since the drive unit 20 can be pushed into the housing 12 manually, the motor 110, the gear mechanism, and the like for connecting the connection portion 92 to the distal end portion of the prefilled syringe 16 are unnecessary. Therefore, the liquid medicine administration device 10 can be simplified.

The connection portion 92 includes the connection needle 102 capable of penetrating the sealing member 60 at the distal end portion of the prefilled syringe 16, and the needle coupling portion 104 that couples the proximal end of the connection needle 102 and the proximal end of the puncture needle 96 to each other. The first connection seal portion 128 seals the connection needle 102. The second connection seal portion 130 seals the sealing member 60. In the liquid medicine administration device 10, the drive unit 20 is pushed into the housing 12, so that the connection needle 102 penetrates the sealing member 60.

According to such a configuration, the connection portion 92 can be easily connected to the distal end portion of the prefilled syringe 16 by allowing the connection needle 102 to penetrate the sealing member 60.

The opening member 124 includes the puncture seal portion 126.

According to such a configuration, by removing the opening member 124 from the housing 12, the puncture seal portion 126 can be opened together when the first connection seal portion 128 and the second connection seal portion 130 are opened.

The adhesive member 14 that can be adhered to skin of a living body is fixed to the outer surface of the housing 12. The opening member 124 is attached to the housing 12 so as to cover at least a part of the adhesive member 14 from the outside.

According to such a configuration, the housing 12 can be fixed to the skin by adhering the adhesive member 14 to the skin after the opening member 124 is removed from the housing 12.

The housing 12 includes the bottom wall portion 28 and the pair of side wall portions 40 protruding from both end portions of the bottom wall portion 28 in the width direction orthogonal to the axial direction of the prefilled syringe 16. The adhesive member 14 is fixed to the outer surface of the bottom wall portion 28 of the housing 12 and extends in a sheet shape. The opening member main body 125 has the base portion 132 extending along the shapes of the bottom wall portion 28 and the pair of side wall portions 40 of the housing 12. The opening member 124 is attached to the housing 12 by engaging the both end portions of the base portion 132 with the pair of side wall portions 40.

According to such a configuration, it is possible to suppress unintentional disengagement of the opening member 124 from the housing 12.

The first opening portion 34 and the second opening portion 36 are formed in the bottom wall portion 28 of the housing 12. The first opening portion 34 is a hole for causing the puncture needle 96 to protrude outward of the housing 12. The opening member main body 125 has the insertion portion 140 to be inserted into the second opening portion 36. The insertion portion 140 is positioned between the prefilled syringe 16 and the connection portion 92 in the state of being inserted into the second opening portion 36. The puncture seal portion 126 is fixed to the base portion 132 such that the first opening portion 34 is sealed in the initial state. The first connection seal portion 128 is fixed to the insertion portion 140.

According to such a configuration, in a state where the opening member 124 is mounted to the housing 12, the puncture seal portion 126 can seal the puncture needle 96, and the first connection seal portion 128 can seal the connection portion 92.

The opening member main body 125 is formed by integrally molding the base portion 132 and the insertion portion 140.

According to such a configuration, the configuration of the sterilization seal structure 22 can be simplified.

The opening member 124 is configured by a resin material.

According to such a configuration, the opening member 124 having appropriate strength can be easily manufactured.

The second connection seal portion 130 is formed in a film shape and is joined to the insertion portion 140.

According to such a configuration, the configuration of the sterilization seal structure 22 can be simplified.

The second connection seal portion 130 extends in one direction. The slit 143 through which the second connection seal portion 130 is inserted is formed in the base portion 132. The one end portion of the second connection seal portion 130 is joined to the distal end portion of the prefilled syringe 16. The other end portion of the second connection seal portion 130 is joined to the outer surface 132b of the base portion 132 on the side opposite to the surface (inner surface 132a) on which the insertion portion 140 is provided.

According to such a configuration, the second connection seal portion 130 can be integrated with the opening member 124 after sterilizing the prefilled syringe 16 and the second connection seal portion 130 in a state where the second connection seal portion 130 is joined to the distal end portion of the prefilled syringe 16.

In the initial state, the second connection seal portion 130 includes the first extension portion 154, the folded portion 156, and the second extension portion 158. The first extension portion 154 extends from the one end portion of the second connection seal portion 130 in the direction opposite to the second opening portion 36. The folded portion 156 is positioned at the extension end portion of the first extension portion 154. The second extension portion 158 extends from the folded portion 156 toward the second opening portion 36.

According to such a configuration, by removing the opening member 124 from the housing 12, the portion of the second connection seal portion 130 joined to the distal end portion of the prefilled syringe 16 can be gradually peeled off from the position close to the first extension portion 154. With this configuration, the second connection seal portion 130 can be smoothly peeled off from the distal end portion of the prefilled syringe 16.

Each of the puncture seal portion 126 and the first connection seal portion 128 is configured by a silicone material.

According to such a configuration, the puncture seal portion 126 configured by the silicone material can efficiently seal the puncture needle 96, and an antibacterial effect can be enhanced. Furthermore, the first connection seal portion 128 configured by the silicone material can efficiently seal the connection needle 102, and the antibacterial effect can be enhanced.

The method for manufacturing the liquid medicine administration device 10 according to the present embodiment includes the first sterilization process, the second sterilization process, the mounting process, and the insertion process. In the first sterilization process, the opening member main body 125 to which the puncture seal portion 126 and the first connection seal portion 128 are fixed is mounted to the housing 12, and the housing 12, the puncture unit 18 disposed inside the housing 12, the puncture seal portion 126, the first connection seal portion 128, and the opening member main body 125 are sterilized. In the second sterilization process, the prefilled syringe 16 and the second connection seal portion 130 are sterilized in a state where the second connection seal portion 130 is joined to the distal end portion of the prefilled syringe 16. In the mounting process, the prefilled syringe 16 subjected to the second sterilization process is mounted to the drive unit 20. In the insertion process, the prefilled syringe 16 and the drive unit 20 are inserted into the housing 12.

According to such a method, since the prefilled syringe 16 subjected to the sterilization processing is mounted to the drive unit 20, the liquid medicine administration device 10 can be manufactured without sterilizing the drive unit 20. With this configuration, it is possible to prevent a failure from occurring in an electronic component or the like of the drive unit 20 due to the sterilization.

The liquid medicine administration device 10 is not limited to the configuration described above. The opening member 124 does not have to be rotatable about the first engagement portion 136. In this case, for example, the opening member 124 may be configured to be removable from the housing 12 by pushing and expanding the both end portions of the base portion 132 (pressing so as to widen an interval between the both end portions of the base portion 132). The puncture seal portion 126 may be provided separately from the opening member 124.

Note that the present invention is not limited to the above disclosure, and various configurations can be adopted without departing from the gist of the present invention.

## Claims

1. A liquid medicine administration device comprising:
a prefilled syringe;
a puncture unit including a puncture needle capable of puncturing skin of a living body, and a connection portion coupled to a proximal end of the puncture needle and connectable to a distal end portion of the prefilled syringe;
a housing that accommodates the puncture unit and the prefilled syringe;
a drive unit for pressing a gasket of the prefilled syringe in a distal end direction; and
a sterilization seal structure for holding the puncture unit and the prefilled syringe in a sterilization state in an initial state,
the connection portion being separated from the distal end portion of the prefilled syringe in the initial state,
wherein the sterilization seal structure includes:
a puncture seal portion for sealing the puncture needle;
a first connection seal portion for sealing the connection portion;
a second connection seal portion for sealing the distal end portion of the prefilled syringe; and
an opening member including the first connection seal portion, the second connection seal portion, and an opening member main body,
the opening member is attached to the housing such that the first connection seal portion and the second connection seal portion are openable,
the prefilled syringe is fixed to the drive unit,
the drive unit is slidably inserted into the housing together with the prefilled syringe in the distal end direction of the prefilled syringe,
the drive unit protrudes from the housing in a direction opposite to the distal end direction of the prefilled syringe in the initial state, and
by pushing, in a state where the first connection seal portion and the second connection seal portion are opened, the drive unit into the housing in the distal end direction of the prefilled syringe such that a protruding length of the drive unit from the housing is shortened, the connection portion is connected to the distal end portion of the prefilled syringe.

2. The liquid medicine administration device according to claim 1, wherein
the connection portion includes:
a connection needle capable of penetrating a sealing member at the distal end portion of the prefilled syringe; and
a needle coupling portion that couples a proximal end of the connection needle and a proximal end of the puncture needle to each other,
the first connection seal portion seals the connection needle,
the second connection seal portion seals the sealing member, and
the connection needle penetrates the sealing member by pushing the drive unit into the housing.

3. The liquid medicine administration device according to claim 1, wherein
the opening member includes the puncture seal portion.

4. The liquid medicine administration device according to claim 1**,** wherein
an adhesive member adherable to the skin of the living body is fixed to an outer surface of the housing, and
the opening member is attached to the housing so as to cover at least a part of the adhesive member from outside.

5. The liquid medicine administration device according to claim 4, wherein
the housing includes:
a bottom wall portion; and
a pair of side wall portions protruding from both end portions of the bottom wall portion in a width direction orthogonal to an axial direction of the prefilled syringe,
the adhesive member is fixed to an outer surface of the bottom wall portion of the housing and extends in a sheet shape,
the opening member main body includes a base portion extending along shapes of the bottom wall portion and the pair of side wall portions of the housing, and
the opening member is attached to the housing by engaging both end portions of the base portion with the pair of side wall portions.

6. The liquid medicine administration device according to claim 5, wherein
a first opening portion and a second opening portion are formed in the bottom wall portion of the housing,
the first opening is a hole through which the puncture needle protrudes outward from the housing,
the opening member main body includes an insertion portion to be inserted into the second opening,
the insertion portion is positioned between the prefilled syringe and the connection portion in a state of being inserted into the second opening portion,
the puncture seal portion is fixed to the base portion such that the first opening portion is sealed in the initial state, and
the first connection seal portion is fixed to the insertion portion.

7. The liquid medicine administration device according to claim 6, wherein
the opening member main body is formed by integrally molding the base portion and the insertion portion.

8. The liquid medicine administration device according to claim 7, wherein
the opening member main body is configured by a resin material.

9. The liquid medicine administration device according to claim 8, wherein
the second connection seal portion is formed in a film shape and is joined to the opening member main body.

10. The liquid medicine administration device according to claim 9, wherein
the second connection seal portion extends in one direction,
a slit through which the second connection seal portion is inserted is formed in the base portion,
one end portion of the second connection seal portion is joined to the distal end portion of the prefilled syringe, and
the other end portion of the second connection seal portion is joined to an outer surface of the base portion on a side opposite to a surface on which the insertion portion is provided.

11. The liquid medicine administration device according to claim 10, wherein
in the initial state, the second connection seal portion includes:
a first extension portion that extends in a direction opposite to the second opening portion from the one end portion of the second connection seal portion;
a folded portion positioned at an extension end portion of the first extension portion; and
a second extension portion that extends from the folded portion toward the second opening portion.

12. The liquid medicine administration device according to claim 1, wherein
each of the puncture seal portion and the first connection seal portion is configured by a silicone material.

13. A method for manufacturing the liquid medicine administration device according to any one of claims 1 to 12, the method comprising:
a first sterilization process of mounting the opening member main body to which the puncture seal portion and the first connection seal portion are fixed to the housing, and sterilizing the housing, the puncture unit disposed inside the housing, the puncture seal portion, the first connection seal portion, and the opening member main body;
a second sterilization process of sterilizing the prefilled syringe and the second connection seal portion in a state where the second connection seal portion is joined to the distal end portion of the prefilled syringe;
a mounting process of mounting the prefilled syringe subjected to the second sterilization process to the drive unit; and
an insertion process of inserting the prefilled syringe and the drive unit into the housing.
